# EUROPEAN PATENT SPECIFICATION

(11) **EP 2 127 467 B1**
(45) Date of publication and mention of the grant of the patent: **28.10.2015**
(21) Application number: 06841011.7
(22) Date of filing: 18.12.2006
(51) Int. Cl.: A61F 11/08, H04R 1/10, H04R 25/00

(54) **ACTIVE HEARING PROTECTION SYSTEM**
SYSTEM ZUM AKTIVEN GEHÖRSCHUTZ
SYSTÈME DE PROTECTION AUDITIVE ACTIVE

(43) Date of publication of application: 02.12.2009
(73) Proprietor: Sonova AG, 8712 Stäfa (CH)
(72) Inventor: PLATZ, Rainer, 2013 Colombier (CH); HAUTIER, Olivier, 2065 Savagnier (CH)
(74) Representative: Schwan Schorer & Partner mbB
(86) International application number: PCT/EP2006/012201
(87) International publication number: WO 2007/082579

(56) References cited:
- EP-A- 0 483 701
- EP-A- 1 640 972
- EP-A- 1 674 057
- US-A1- 2004 136 522

## Description

The present invention relates to an active haring protection system comprising a left and a right earplug, each being adapted for providing for an acoustic attenuation of at least 10 dB averaged over the audible frequency range and comprising an active unit with a microphone for capturing audio signals from ambient sound and a loudspeaker.

US 4,677,678 relates to a hearing protection system comprising two earmuffs or earplugs comprising each a microphone and a speaker. The system comprises two audio channels sharing a common sound level limiting unit.

US 2002/0080979 A1 relates to a hearing protection system comprising two earplugs into which an active unit comprising each a microphone and a speaker is inserted. The gain as a function of the sound input level is a three-segment piece-wise linear function including a first section providing maximum expansion up to a first knee point for maximum noise reduction, a second section providing less expansion up to a second knee point for less noise reduction, and a third section providing minimum or no expansion for input signals with high signal-to-noise-ratio (SNR) to minimise distortion.

EP 1 674 061 A1 relates to a hearing protection system comprising two earplugs including an active unit comprising a microphone and a speaker, wherein the audio signal processing mode is controlled by sound classification including level analysis. If the acoustic noise level is high, the gain may be significantly reduced or even completely eliminated. The system includes a transparent gain setting if acoustic noise is low, and a close speech mode if close speech in acoustic noise has been detected.

EP 1 674 057 A1, EP 1 615 468 A1 and DE 101 17 705 A1 relate to hearing protection systems comprising a microphone, an audio signal processing unit and a speaker at each ear, wherein in addition a wireless interface for an external audio source, such as a mobile phone or a remote active hearing protection system used by another person, is provided.

Hearing protection systems provided with an interface for wireless audio input from a remote audio signal source, but not having microphones at ear level, are known, for example, from EP 1 674 059 A1, GB 2 373 951 A, US 5,426,719 and US 2003/0059071 A1.

EP 1 653 773 A2 relates to a hearing aid which is capable of automatically selecting, for example according to the SNR or according to the audio signal level, the audio signal input from one of a plurality of audio signal sources. The audio signal sources may be a T-coil receiver, a frequency modulation (FM) radio receiver or a direct audio input. The hearing aid also comprises a classifier for selecting the audio signal processing mode of the hearing aid according to the analysis of the auditory scene. A similar hearing aid is described in EP 1 443 803 A2.

US 5,721,783 relates to a hearing instrument comprising two ear-pieces with a microphone and a speaker and a remote audio signal processing unit wirelessly connected to the ear-pieces. Audio signals from a phone can be provided via the remote audio signal processing unit to the ear-pieces. A switch is provided for switching between the microphone signals and the phone signals as the audio input to audio signal processing unit. The system may comprise active noise cancelling capability.

US 2004/0136522 A1 relates to a headset which comprises at each ear an active noise reduction (ANR) arrangement comprising a microphone and a speaker for generating anti-noise. Further, the headset comprises at each ear a communication loudspeaker provided with audio signals from a primary communication device and/or a secondary communication device. In addition, the headset comprises a boom microphone whose audio signal may be provided, after pre-amplification in a controller, to the primary and secondary communication device. The controller, which acts a central unit utilized by both sides of the headset, comprises a device detector, which senses the presence of the secondary communication unit, and a communication priority module for weighting of the audio signals from the primary communication device and the secondary communication device.

It is an object of the invention to provide for an active hearing protection system which provides for a particularly comfortable and flexible communication function. It is a further object to provide for a corresponding method for providing hearing protection to a user.

According to the invention, these objects are achieved by an active hearing protection system as defined in claim 1 and a method as defined in claim 15, respectively. The invention is beneficial in that, by providing for a central unit having a digital audio signal processing unit having at least two channels and by automatically controlling the audio signal processing unit depending on whether the presence of an audio signal at the audio input of the audio signal processing unit has been detected or not, external audio signals can be combined for communication purposes in a particularly comfortable and flexible manner with the audio signal captured by the microphones of the system.

Preferred embodiments of the invention are defined in the dependent claims.

In the following, examples of the invention will be illustrated by reference to the attached drawings, wherein:
- Fig. 1: is a schematic view of an example of an active hearing protection system according to the invention;
- Fig. 2: is a block diagram of the electronic components of the system of Fig. 1;
- Fig. 3: is a block diagram of the system wherein operation in the communication mode is illustrated; and
- Fig. 4: is a diagram showing an example of the acoustic gain model of the system.

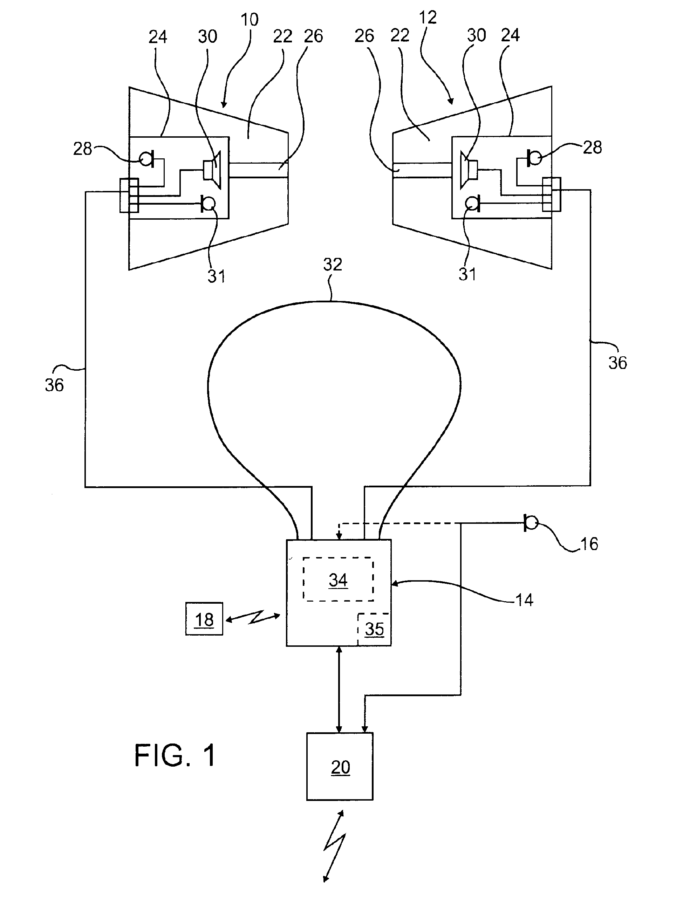

The active hearing protection system shown in Fig. 1 comprises a left ear earplug 10, a right ear earplug 12, a central unit 14, a boom microphone 16 and a remote control unit 18. The central unit 14 is connected to a communication device 20.

Preferably the hearing protection earplugs 10, 12 have a hard shell with an elasticity from shore D85 to shore D65, which is customized, i.e. it has an outer shape according to the individual measured inner shape of the user's outer ear and ear canal. The hard shell 22 may be manufactured by layer-by-layer laser sintering of a powder material, for example, polyamide powder or by laser stereo-lithography or photo-polymerization. An overview regarding such additive layer-by-layer build-up processes for manufacturing customized shells of hearing devices can be found, for example, in US 2003/0133583 A1 or US 6,533,062 B1. The inner shape of the user's ear canal and outer ear can be measured, for example, by taking an impression which then undergoes laser scanning or by direct laser scanning of the ear. Preferably the hard shell 22 is designed such that it provides for an acoustic attenuation of at least 10 dB averaged over the audible frequency range when worn by the user.

The shell 22 has a receptacle into which an active unit 24 can be inserted, preferably in a releasable manner. The shell 22 is provided with a sound channel 26 through which the active unit 24 is acoustically connected to the ear canal. The active unit 24 comprises a microphone 28 for capturing audio signals from ambient sound and a loudspeaker 30 for providing audio signals into the user's ear canal via the sound channel 26. Such earplugs comprising an active unit are described, for example, in EP 1 674 059 A1.

The central unit 14 is to be worn at the user's body, e.g. by a loop 32 around the user's neck. The central unit 14 comprises an audio signal processing unit 34 for receiving and processing the audio signals captured by the microphones 28 and for supplying the loudspeakers 30 with audio signals to be reproduced to the user's ear. To this end, the active units 24 are connected to the central unit 14 via cable connections 36. The remote control unit 18 is connected to the central unit 14 via a wireless link in order to serve as a user interface.

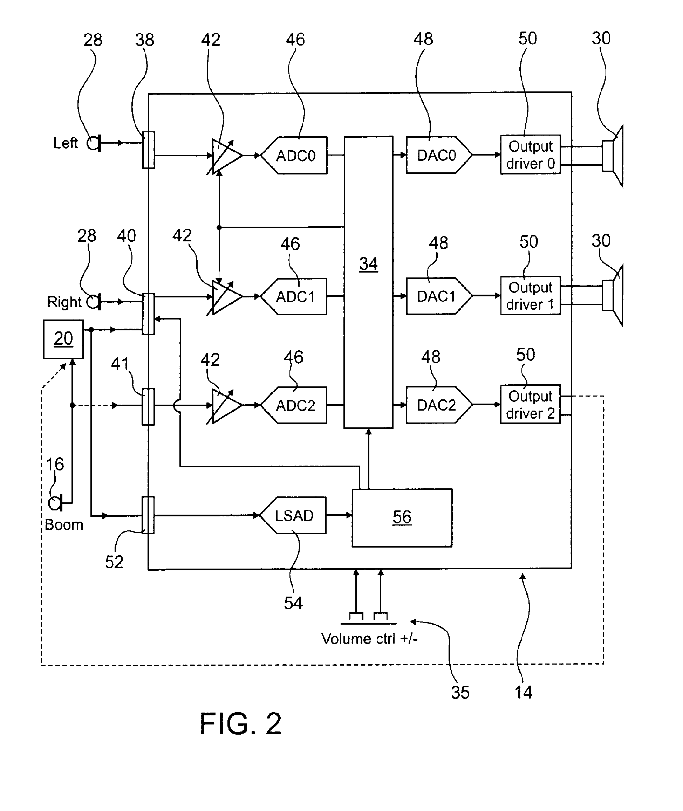

The boom microphone 16 may be connected to the central unit in order to supply audio signals captured from the user's voice (dashed lines in Figs. 1 and 2). Alternatively, it may be connected directly to the communication device 20. The communication device 20, for example, may be a mobile phone or a FM radio device, such as a walkie-talkie. Usually the communication device 20 and the central unit 14 will be connected by wires, although connection by a wireless link is conceivable.

In order to act as an active hearing protection system, audio signals captured from ambient sound by the microphones 28 are provided, after processing in the audio signal processing unit 34, to the speakers 30 in order to provide sound impinging on the left ear to the left ear canal and sound impinging on the right ear to the right ear canal despite the acoustic attenuation provided by the shell 22 for relatively low sound pressure levels (for high sound pressure levels the gain will be progressively reduced in order to provide for a hearing protection function). Further, audio signals received from the communication device 20 are also to be provided to the user's ear via the central unit 14 and the speakers 30. In addition, if bidirectional communication is desired, the user's voice can be captured by the boom microphone 16 and/or the microphones 28, 31 and will be supplied to the communication device 20.

The structure and the functionality of the central unit 14 will be explained in more detail by reference to Figs. 2 to 4. The central unit 14 comprises an input 38 for the left ear microphone 28, an input 40 for the right ear microphone 28 and the audio signals from the communication device 20 and optionally an input 41 for the boom microphone 16. Each of these channels is provided with a pre-amplifier 42 which preferably is adjustable. The pre-amplified signal is digitized in an analogue-to-digital converter 46 before it is supplied to the audio signal processing unit 34, which has at least two outputs for outputting corresponding the processed digital audio signals to a digital-to-analogue converter 48, with the respective analogue signal being provided to an output driver 50 driving the left ear speaker 30 and the right ear speaker 30, respectively. A third output comprising a digital-to-analogue converter 48 and an output driver 50 may be provided for supplying audio signals from the audio signal processing unit 34 to the communication device 20.

The central unit 14 comprises a further audio input 52 which is connected in the same manner as the input 40 to the communication device 20 and hence receives the same audio signals. The audio signals received at the input 52 are provided to a low speed analogue-to-digital converter 54, with the digitized signals being provided to a detector 56 for detecting the presence of an audio signal from the communication device 20 at the audio input 52. On the one hand, the detector 56 supplies a status signal to the audio signal processing unit 34 in order to select the audio signal processing mode depending on whether there is an audio signal from the communication device 20 or not. On the other hand, the detector 56 also acts on the audio input 40 which can be switched between the microphone 28 and the communication device 20 depending on whether an audio signal from the communication device 20 is present or not.

If no audio signal from the communication device 20 is detected by the detector 56, the audio signal processing unit 34 operates in an "ambient mode" in which the right ear microphone 28 is selected as the audio signal source to the input 40 rather than the communication device 20. In the ambient mode the audio signal processing unit 34 primarily acts as a stereo device, i.e. the audio signals from the right ear microphone 28 are supplied essentially to the right ear speaker 30, and the audio signals from the left ear microphone 28 are essentially supplied to the left ear speaker 30. However, there may be some transfer of audio signals between the two channels in order to enable binaural audio signal processing. Examples of binaural audio signal processing can be found in EP 1 320 281 A2, WO 99/43185 or US 2004/02 52852 A1. Apart from such binaural processing, both channels usually will be processed in the same manner, if the user is a normal hearing person. If, however, the user suffers from asymmetric hearing loss, also the audio signal processing in the "ambient mode" will be asymmetric in order to compensate for the hearing loss as far as possible.

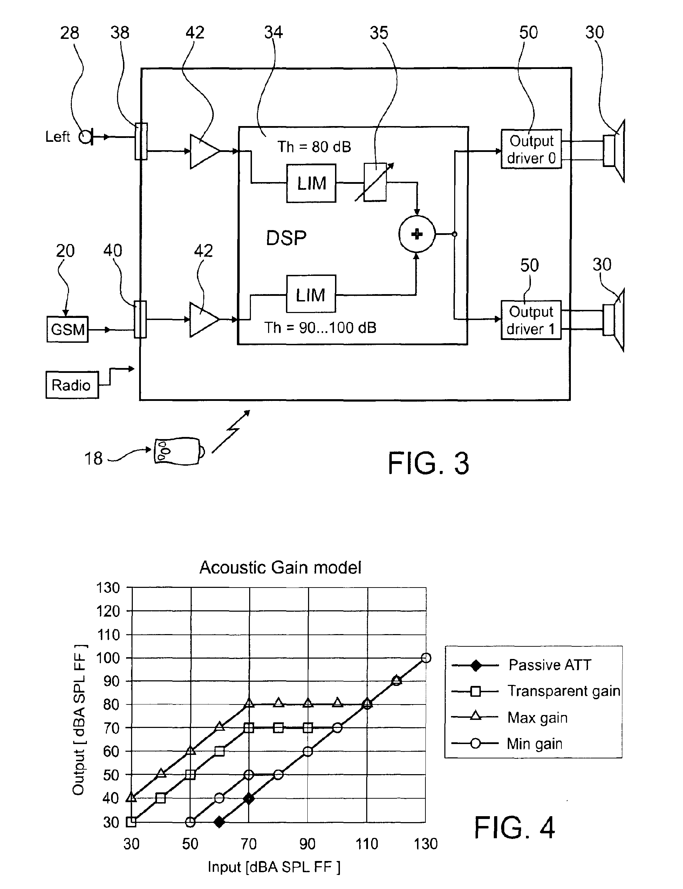

In Fig. 4 an example of the acoustic gain model implemented in the audio signal processing unit 34 is shown, wherein the output free field sound pressure level (SPL FF) is given as a function of the input SPL FF. In the example shown in Fig. 4 it is assumed that the shell 22 provides for an attenuation of 30 dB. The curve with the diamonds represents the situation in which the gain is set to zero (i.e. no sound from the speakers 30). On this curve, the hearing protection system acts as a passive system.

The curve with the squares represents the situation in which the system has a "transparent" gain, i.e. below the knee point (which is set to 70 dBA) the user has the same sound feeling as if he was not wearing the earplugs 10, 12. Different gain settings are applied below and above the knee point (which is set to 70 dBA in the example of Fig. 4). Below this knee point the gain is constant, resulting in a linear curve. Above the knee point, i.e. for higher input levels, the gain is progressively reduced so that the output level remains constant. For even higher input levels the system will act as a passive hearing protector.

The system also comprises a manual volume control which is implemented on the central unit 14 or, alternatively or additionally, in the remote control unit 18. By actuating the volume control, the value of the constant gain applied in the linear region below the knee point can be adjusted between a minimum value (circles in Fig. 4) and a maximum value (triangles in Fig. 4). When the maximum gain is applied, the maximum output free field sound pressure level is limited to 80 dBA SPL FF in the example of Fig. 4. 85 dBA SPL FF correspond to the maximum noise level tolerable for 8 hours a day according to Directive 2003/10/EC. Since the open ear gain (OEG) is around 10 dB, the sound pressure level in the ear canal is around 10 dB higher than the free-field values given in Fig. 4.

In Fig. 4 the gain model is shown for one frequency. The gain model to be applied by the audio signal processing unit 34 may be frequency-dependant in order to compensate for the frequency-dependency of the acoustic attenuation provided by the shell 22 and/or a hearing loss of the user.

Beyond the volume control interface, the central unit 14 may comprise manual user interfaces such as e.g. on/off, audio signal processing mode (program) change, push-to-talk (PTT), call accept/reject or voice dialing. These manual control interfaces are designated by 35 in Figs. 1 and 2. The same commands or a subset thereof may be provided on the remote control unit 18.

The acoustic gain model may be automatically selected according to the result of an auditory scene analysis based on the audio signals provided by the microphones 28.

According to an example not covered by the present invention, once an audio signal from the communication device 20 is detected by the detector 56, the central unit 14 will switch into a "communication mode" in which the input from the right ear microphone 28 is replaced by the audio signals provided by the communication device 20. Whereas in the ambient mode - apart from compensation of an asymmetric hearing loss - usually the same gain model is applied to both channels, in the communication mode preferably a different gain model now is applied to that channel to which the audio signals from the communication device 20 are provided to. In this "communication channel" the maximum output free field sound pressure level may be shifted to higher values, for example in such a manner that an output level of 100 dBA SPL FF is allowed (rather than 80 dBA SPL FF allowable for the channel of the left ear). This level limiting function in both channels is labelled "LIM" ("limiter")in Fig. 3. In the communication mode the volume control will act only on the "ambient channel", i.e. the left ear microphone channel. After having undergone respective audio signal processing, the two channels may be combined and then may be supplied as a mono signal to both loudspeakers 30.

Such an example of operation of the system in the communication mode is schematically shown in Fig. 3 (the right ear microphone 28 is omitted in Fig. 3, since it is blocked by action of the detector 56 on the input 40). According to this example in the communication mode a mixture of audio signals representative of the ambient sound as captured by the left ear microphone 28 and the audio signals provided by the communication device 20 is provided to both ears. The user can adjust the relative level of the "ambient channel" and the "communication channel" by actuating the volume control on the central unit 14 or via the remote control unit 18, which in the communication mode acts only on the "ambient channel".

The volume of the communication channel may be adjusted on the communication device 20.

In case that the audio signals provided by the communication device 20 are speech signals the "communication channel" may undergo some speech enhancement filtering in order to improve intelligibility of the speech. Enhancement of the intelligibility of the voice in the "communication channel" can be achieved, for example, by reducing the audio band width and/or emphasizing speech frequencies (300 Hz to 3.4 kHz). However, such filtering also my be applied to the "ambient channel" or to both channels in the ambient mode if speech signals are received via the microphones 28. The decision that speech is present may be made by performing auditory scene analysis in the audio signal processing unit 34 based on the audio signals captured by the microphones 28.

According to the invention, in the communication mode the audio signals from the communication device 20 may be mixed to at least one of the audio signals captured by the left ear microphone 28 and the right ear microphone 28, with, as in the ambient mode, the audio signals captured by the left ear microphone 28 being provided to the left ear speaker and the audio signals from the right ear microphone 28 being provided to the right ear speaker 30. The mixing ratio may be different for the two channels. In this case the central unit would be equipped with a further fast analogue-to-digital converter, so that the audio signals from the communication device 20 could be processed in parallel to the audio signals from the left ear microphone 28 and the right ear microphone 28.

Preferably the interface between the communication device 20 and the central unit 14 is bidirectional so that not only audio signals can be supplied from the communication device 20 to the central unit 14 but also audio signals and/or command signals can be sent from the central unit 14 to the communication device 20. For example, the user's voice may be captured by the boom microphone 16 and/or the microphones 28 in order to be supplied to the communication device 20 via the third output (dashed lines).

The boom microphone 16 may be omitted if the central unit 14 is capable of performing blind source separation (BSS) on the audio signals provided by the microphones 28, 31 in order to separate the user's voice from background noise. Typically, the inner microphone 31 is required on only one of both sides. Preferably, each active unit 24 of the earplugs 10, 12 in this case may include a second microphone 31 which is acoustically oriented towards the ear canal in order to capture primarily the user's voice, whereas the microphone 28 is acoustically oriented towards ambience in order to capture primarily ambient sound.

Alternatively, one or two microphones 31 on the right and/or the left side may pick up the user's voice transferred by bone conduction to the ear canal (in-ear voice pickup).

Alternatively or in addition, the central unit 14 may be capable of performing acoustic beamforming on the audio signals captured by the microphones 28 in order to separate the user's voice from ambient sound/background noise.

The central unit 14 and/or remote control unit 18 preferably comprises a PTT (Push to Talk) element operable by the user to control transmission of audio signals, representing the user's voice, via the communication device 20.

The central unit 14 and/or remote control unit 18 also may comprise a call accept/call reject element and a voice dialling activation element for the event that the communication device 20 is a mobile phone.

The communication device 20 also may serve to supply power to the central unit 14. Alternatively, the central unit 14 may be provided with a primary or rechargeable battery (not shown in Figs. 1 to 3).

The audio signals representing the user's voice captured by the microphones 28 and/or the boom microphone 16 may be supplied not only to the communication device 20 but also to some extent to the right ear and left ear speaker 30 in order to reduce the occlusion effect caused by the acoustic attenuation of the shell 22.

The central unit 14 may be capable of user preference learning in order to learn and store the preferred manual settings by the user.

Preferably, the audio signal processing unit 34 is realized as a single digital signal processing (DSP) chip.

The central unit 14 also may comprise a noise dosimeter functionality, with the sound exposure of the user being estimated from the level of the audio signals captured by the left ear and right ear microphones 28 or 31, which is integrated over a given time period. The central unit 14 may comprise an alarm function wherein the user is alerted via at least one of the speakers 30 when a given maximum noise dose has been reached. Alternatively or in addition, the central unit 14 may comprise a protection function wherein the gain applied in the audio signal processing unit 34 is reduced when a given maximum noise dose has been reached.

## Claims

1. An active hearing protecting system comprising a right earplug (10) to be worn at least in part in the right ear canal of a user and a left earplug (12) to be worn at least in part in the left ear canal of the user and a central unit (14) to be worn at the user's body and comprising a digital audio signal processing unit (34) which has at least two channels and which can be connected via an audio input (40) to an external audio signal source (20),
wherein each earplug (10, 12) is adapted for providing for an acoustic attenuation of at least 10 dB averaged over the audible frequency range and comprises an active unit (24) comprising a microphone (28) for capturing audio signals from ambient sound and a loudspeaker (30) which are connected to the audio signal processing unit (34),
wherein the central unit comprises a detector (56) for detecting the presence of an audio signal at the audio input (40, 52),
and wherein the central unit (14) is adapted to control the audio signal processing unit (34) in such a manner that, during times when no presence of an audio signal at the audio input (40, 52) is detected, the audio signal processing unit (34) is operated in an ambient mode in which the audio signals captured by the left ear microphone (28) are provided essentially to the left ear loudspeaker (30) and the audio signals captured by the right ear microphone (28) are provided essentially to the right ear loudspeaker (30), and during times when the presence of an audio signal at the audio input (40, 52) is detected, the audio signal processing unit (34) is operated in a communication mode in which the audio signals captured by the left ear microphone (28) are provided to the left ear loudspeaker (30) and the audio signals captured by the right ear microphone (28) are provided to the right ear loudspeaker (30), wherein the audio signals received via the audio input (40, 52) are mixed with at least one of the audio signals captured by the left ear microphone (28) and the audio signals captured by the right ear microphone (28) in order to be provided to at least one of the left ear (30) loudspeaker and the right ear loudspeaker (30), and wherein at least one parameter used for audio signal processing in the audio signal processing unit (34) is different for the ambient mode and the communication mode for at least one of the channels, and wherein the parameter is the gain as a function of the input level.

2. The system of claim 1, wherein the mixing ratio is different for mixing with the audio signals captured by the left ear microphone (28) and for mixing with the audio signals captured by the right ear microphone (28).

3. The system of one of the preceding claims, wherein both in the ambient mode and in the communication mode for the two channels of the audio signal processing unit (34) the gain is progressively reduced above a given threshold value of the input level, wherein the gain is essentially independent of the input level for input levels below the threshold value, wherein for input levels below the threshold value the gain of at least one of the channels is adjustable by a manual volume control (18), wherein in the ambient mode the gain of both channels is adjustable in parallel by the manual volume control (18), wherein in the communication mode only the gain of that channel carrying primarily the audio signals captured by at least one of the left ear microphone (28) and right ear microphone (28) is adjustable by the manual volume control (18), wherein in the communication mode the maximum acoustic output level is different for the two channels, wherein in the ambient mode the gain function of the audio signal processing unit (34) can be selected manually or is automatically selected based on the result of an auditory scene analysis, wherein the central unit (14) comprises a bidirectional interface to a wireless communication device (20), which communication device is for providing audio signals to the audio input (40, 52), wherein the system comprises means (16, 28) for capturing audio signals to be supplied to the communication device (20) and wherein the system comprises a remote control unit (18) adapted for manual control of the communication device (20), wherein the central unit (14) comprises a bidirectional interface to a wireless communication device (20), which communication device is for providing audio signals to the audio input (40, 52), wherein the system comprises means (16, 28) for capturing audio signals to be supplied to the communication device (20) and wherein the central unit (14) comprises a user interface for manual control of the communication device (20), and wherein the bidirectional interface is wireless.

4. The system of claim 3, wherein the means for capturing audio signals is a boom microphone (16) which is to be worn by the user to capture the user's voice and which is electrically connected to the communication device directly or via said interface.

5. The system of claim 3, wherein the means for capturing audio signals is at least one of the left ear microphone (28) and the right ear microphone (28) and/or at least one inner microphone (31) acoustically oriented inwardly towards the ear canal.

6. The system of claim 5, wherein a BSS unit is included in the audio signal processing unit (34) in order to have the audio signals captured by at least one of the left ear microphone (28) and the right ear microphone (28) and/or at least one inner microphone (31) undergo a BSS algorithm in order to separate the user's voice from ambient sound.

7. The system of claim 5, wherein the audio signal processing unit (34) is capable of performing acoustic beamforming on the audio signals captured by the left ear microphone (28) and the right ear microphone (28) in order to separate the user's voice from ambient sound, and wherein the remote control unit (18) is a handheld unit connected to the central unit (34) by a wireless link.

8. The system of claim 3, wherein the central unit (14) and/or the remote control unit (18) comprises a Push-to-talk element (35) for controlling transmission of the audio signals supplied by the audio signal capturing means (16, 28, 31) by the communication device (20).

9. The system of claim 3, wherein the remote control unit (18) comprises a volume control element for controlling the gain applied by the audio signal processing unit (34).

10. The system of claim 3, wherein the communication device (20) is a mobile phone, and wherein the remote control unit (18) comprises a call accept / call reject element and a voice dialing activation element.

11. The system of claim 3, wherein the communication device (20) is a mobile phone, and wherein the central unit (14) comprises a call accept / call reject element and a voice dialing activation element, wherein power is supplied from the communication device (20) to the central unit (14) via the bidirectional interface, wherein the audio signal processing unit (34) in the ambient mode is adapted to enhance by filtering the user's voice in the audio signals captured by the left ear microphone (28) and the right ear microphone (28) or in the audio signals captured by a boom microphone (16), with the filtered audio signals being provided to the right ear and left ear loudspeaker (30) in order to reduce occlusion effect, wherein the central unit (14) is capable of user preference learning in order to learn and store the preferred manual settings by the user, wherein the audio signal processing unit (34) is a single DSP chip, wherein the central unit (14) comprises a noise dosimeter functionality, with the sound exposure of the user being estimated from the level of the audio signals captured by the left ear and/or right ear microphone (28) and/or by at least one inner microphone (31) acoustically oriented inwardly towards the ear canal and integrated over a given time period, wherein the central unit (14) comprises an alarm function wherein the user is alerted via at least one of the left ear and right car (30) loudspeaker when a given maximum noise dose has been reached, wherein the central unit (14) comprises a user protection function wherein the gain applied in the audio signal processing unit (34) is reduced when a given maximum noise dose has been reached, wherein the gain applied by the audio signal processing unit (34) in at least one of the ambient mode and the communication mode is frequency-dependent in order to compensate for at least one of the frequency dependence of the acoustic attenuation provided by the earplugs (10, 12) and a hearing loss of the user, and wherein the gain applied by the audio signal processing unit (34) in at least one of the ambient mode and the communication mode is frequency-dependent in order to enhance speech intelligibility of the audio signals received via the audio input (40, 52).

12. The system of one of the preceding claims, wherein in the ambient mode one of a plurality of audio signal processing modes of the audio signal processing unit (34) is automatically selected according to the result of an auditory scene analysis.

13. The system of one of the claims 1 to 11, wherein in the ambient mode one of a plurality of audio signal processing modes of the audio signal processing unit (34) can be manually selected on the central unit (14) or via a remote control unit (18).

14. The system of one of the preceding claims, wherein each earplug (10, 12) comprises a customized shell (22) into which the active unit (24) is detachably inserted.

15. A method for providing hearing protecting to user, comprising:
wearing a right earplug (10) comprising a microphone (28) and a loudspeaker (30) at least in part in the right ear canal and a left earplug (12) comprising a microphone (28) and a loudspeaker (30) at least in part in the left ear canal in such a manner that each earplug provides for an acoustic attenuation of at least 10 dB averaged over the audible frequency range;
wearing a central unit (14) comprising a digital audio signal processing unit (34) which has at least two channels at the user's body;
connecting an external audio signal source (20) via an audio input (40, 52) to the central unit (14);
detecting whether an audio signal is present at the audio input (40, 52) or not; and
controlling the audio signal processing unit (34) in such a manner that, during times when no presence of an audio signal at the audio input (40, 52) is detected, the audio signal processing unit (34) is operated in an ambient mode in which the audio signals captured by the left ear microphone (28) are provided essentially to the left ear loudspeaker (30) and the audio signals captured by the right ear microphone (28) are provided essentially to the right ear loudspeaker (30), and during times when the presence of an audio signal at the audio input (40, 52) is detected, the audio signal processing unit (34) is operated in a communication mode in which the audio signals captured by the left ear microphone (28) are provided to the left ear loudspeaker (30) and the audio signals captured by the right ear microphone (28) are provided to the right ear loudspeaker (30), and wherein the audio signals received via the audio input (40, 52) are mixed with at least one of the audio signals captured by the left ear microphone (28) and the audio signals captured by the right ear microphone (28) in order to be provided to at least one of the left ear loudspeaker (30) and the right ear loudspeaker (30), and wherein at least one parameter used for audio signal processing in the audio signal processing unit (34) is different for the ambient mode and the communication mode for at least one of the channels, and wherein the parameter is the gain as a function of the input level.

## Patentansprüche

1. Aktives Hörschutzsystem mit einem rechten Ohrstopfen (10), der zumindest zum Teil in dem rechten Gehörgang eines Nutzers zu tragen ist, und einem linken Ohrstopfen (12), der zumindest zum Teil in dem linken Gehörgang des Nutzers zu tragen ist, und einer zentralen Einheit (14), die am Körper des Nutzers zu tragen ist und eine digitale Audiosignalverarbeitungseinheit (34) aufweist, die mindestens zwei Kanäle hat und die über einen Audioeingang (40) mit einer externen Audiosignalquelle (20) verbunden werden kann,
wobei jeder Ohrstopfen (10, 12) ausgebildet ist, um für eine akustische Dämpfung von mindestens 10 dB, gemittelt über den hörbaren Frequenzbereich, zu sorgen, und eine aktive Einheit (24) mit einem Mikrofon (28) zum Auffangen von Audiosignalen aus Umgebungsschall und einen Lautsprecher (30) aufweist, das bzw. der mit der Audiosignalverarbeitungseinheit (34) verbunden ist,
wobei die zentrale Einheit einen Detektor (56) zum Erfassen der Anwesenheit eines Audiosignals an dem Audioeingang (40, 52) aufweist,
und wobei die zentrale Einheit (14) ausgebildet ist, um die Audiosignalverarbeitungseinheit (34) so zu steuern, dass während Zeiten, wenn keine Anwesenheit eines Audiosignals an dem Audioeingang (40, 52) erfasst wird, die Audiosignalverarbeitungseinheit (34) in einem Umgebungsmodus betrieben wird, in welchem die mittels des Linksohr-Mikrofons (28) aufgefangenen Audiosignale im Wesentlichen dem Linksohr-Lautsprecher (30) zugeführt werden und die mittels des Rechtsohr-Mikrofons (28) aufgefangenen Audiosignale im Wesentlichen dem Rechtsohr-Lautsprecher (30) zugeführt werden, und während Zeiten, wenn die Anwesenheit eines Audiosignals an dem Audiosignaleingang (40, 52) erfasst wird, die Audiosignalverarbeitungseinheit (34) in einem Kommunikationsmodus betrieben wird, in welchem die von dem Linksohr-Mikrofon (28 aufgefangenen Audiosignale dem Linksohr-Lautsprecher (30) zugeführt werden und die mittels des Rechtsohr-Mikrofons (28) aufgefangenen Audiosignale dem Rechtsohr-Lautsprecher (30) zugeführt werden, wobei die mittels des Audioeingangs (40, 52) empfangenen Audiosignale mit den mittels des Linkohr-Mikrofons aufgefangenen Audiosignalen und/oder mit den mittels des Rechtsohr-Mikrofons (28) aufgefangenen Audiosignalen gemischt werden, um dem Linksohr-Lautsprecher (30) und/oder dem Rechtsohr-Lautsprecher (30) zugeführt zu werden, und wobei sich mindestens ein für die Audiosignalverarbeitung in der Audiosignalverarbeitungseinheit (34) verwendeter Parameter für den Umgebungsmodus und für den Kommunikationsmodus für mindestens einen der Kanäle unterscheidet, und wobei es sich bei dem Parameter um die Verstärkung als Funktion des Eingangspegels handelt.

2. System gemäß Anspruch 1, wobei sich das Mischverhältnis für das Mischen mit mittels des Linksohr-Mikrofons (28) aufgefangenen Audiosignalen und für das Mischen mit mittels des Rechtsohr-Mikrofons (28) aufgefangenen Audiosignalen unterscheidet.

3. System gemäß einem der vorhergehenden Ansprüche, wobei sowohl in dem Umgebungsmodus als auch in dem Kommunikationsmodus für die beiden Kanäle der Audiosignalverarbeitungseinheit (34) die Verstärkung oberhalb eines gegebenen Schwellwerts des Eingangspegels progressiv reduziert wird, wobei die Verstärkung für Eingangspegel unterhalb des Schwellwerts im Wesentlichen unabhängig von dem Eingangspegel ist, wobei für Eingangspegel unterhalb des Schwellwerts die Verstärkung mindestens eines der Kanäle mittels eines manuellen Lautstärkereglers (18) einstellbar ist, wobei in dem Umgebungsmodus die Verstärkung beider Kanäle mittels der manuellen Lautstärkeeinstellung (18) parallel einstellbar ist, wobei in dem Kommunikationsmodus nur die Verstärkung desjenigen Kanals, welcher hauptsächlich die mittels des Linksohr-Mikrofons (28) und/oder des Rechtsohr-Mikrofons (28) aufgefangenen Audiosignale trägt, mittels der manuellen Lautstärkeerstellung (18) einstellbar ist, wobei sich in dem Kommunikationsmodus der maximale akustische Ausgangspegel für die beiden Kanäle unterscheidet, wobei in dem Umgebungsmodus die Verstärkungsfunktion der Audiosignalverarbeitungseinheit (34) manuell oder automatisch basierend auf dem Ergebnis einer Hörumgebungsanalyse ausgewählt werden kann, wobei die zentrale Einheit (14) eine bidirektionale Schnittstelle mit einem drahtlosen Kommunikationsgerät (20) aufweist, welches vorgesehen ist, um dem Audioeingang (40, 52) Audiosignale zuzuführen, wobei das System Mittel (16, 28) zum Auffangen von Audiosignalen aufweist, die dem Kommunikationsgerät (20) zuzuführen sind, und wobei das System eine Fernsteuereinheit (18) zum manuellen Steuern des Kommunikationsgeräts (20) aufweist, wobei die zentrale Einheit (14) eine bidirektionale Schnittstelle mit dem drahtlosen Kommunikationsgerät (20) aufweist, welches vorgesehen ist, um dem Audioeingang (40, 52) Audiosignale zuzuführen, wobei das System Mittel (16, 28) zum Auffangen von Audiosignalen aufweist, die dem Kommunikationsgerät (20) zuzuführen sind, und wobei die zentrale Einheit (40) eine Benutzerschnittstelle zum manuellen Steuern des Kommunikationsgeräts (20) aufweist, wobei die zentrale Einheit (14) eine bidirektionale Schnittstelle mit dem drahtlosen Kommunikationsgerät (20) aufweist, welches vorgesehen ist, um dem Audioeingang (40, 52) Audiosignale zuzuführen, wobei das System Mittel (16, 28) zum Auffangen von Audiosignalen aufweist, die dem Kommunikationsgerät (20) zuzuführen sind, und wobei die zentrale Einheit (40) eine Benutzerschnittstelle zum manuellen Steuern des Kommunikationsgeräts (20) aufweist, und wobei die bidirektionale Schnittstelle drahtlos ist.

4. System gemäß Anspruch 3, wobei es sich bei den Mitteln zum Auffangen von Audiosignalen um ein Bügelmikrofon (16) handelt, welches von dem Nutzer zu tragen ist, um die Stimme des Nutzers aufzufangen, und welches direkt oder über die Schnittstelle elektrisch mit dem Kommunikationsgerät verbunden ist.

5. System gemäß Anspruch 3, wobei es sich bei den Mitteln zum Auffangen von Audiosignalen um das Linksohr-Mikrofon (28) und/oder das Rechtsohr-Mikrofon (28) und/oder mindestens ein inneres Mikrofon (31) handelt, welches akustisch nach innen zum Gehörgang hin orientiert ist.

6. System gemäß Anspruch 5, wobei die Audiosignalverarbeitungseinheit (34) eine BSS-Einheit beinhaltet, um die mittels des Linksohr-Mikrofons (28) und/oder des Rechtsohr-Mikrofons (28) und/oder mindestens eines inneren Mikrofons (31) aufgefangenen Audiosignale einem BSS-Algorithmus zu unterziehen, um die Stimme des Nutzers von Umgebungsschall zu trennen.

7. System gemäß Anspruch 5, wobei die Audiosignalverarbeitungseinheit (34) in der Lage ist, bezüglich der von dem Linksohr-Mikrofon (28) und dem Rechtsohr-Mikrofon (28) aufgefangenen Audiosignale eine akustische Strahlformung vorzunehmen, um die Stimme des Nutzers von Umgebungsschall zu trennen, und wobei es sich bei Fernsteuereinheit (18) um eine in der Hand zu haltende Einheit handelt, die mit der zentralen Einheit (34) über eine drahtlose Verbindung verbunden ist.

8. System gemäß Anspruch 3, wobei die zentrale Einheit (14) und/oder die Fernsteuereinheit (18) ein Push-to-Talk-Element (35) zum Steuern des Sendens der durch die Audiosignalauffangmittel (16, 28, 31) zugeführten Audiosignale mittels des Kommunikationsgeräts (20) aufweist.

9. System gemäß Anspruch 3, wobei die Fernsteuereinheit (18) ein Lautstärkeeinstellelement zum Steuern der von der Audiosignalverarbeitungseinheit (34) angewandten Verstärkung aufweist.

10. System gemäß Anspruch 3, wobei das Kommunikationsgerät (20) ein Mobiltelefon ist und wobei die Fernsteuereinheit (18) ein Anrufannahme-/Anrufabweisungs-Element und ein Stimmwahlaktivierungselement aufweist.

11. System gemäß Anspruch 3, wobei das Kommunikationsgerät (20) ein Mobiltelefon ist und wobei die Zentraleinheit (14) ein Anrufannahme-/Anrufabweisungs-Element und ein Stimmwahlaktivierungselement aufweist, wobei die zentrale Einheit (14) über die bidirektionale Schnittstelle von dem Kommunikationsgerät (20) mit Strom versorgt wird, wobei die Audiosignalverarbeitungseinheit (34) ausgebildet ist, um im Umgebungsmodus die Stimme des Nutzers mittels Filtern in den von dem Linksohr-Mikrofon (28) und dem Rechtsohr-Mikrofon (28) aufgefangenen Audiosignalen oder in den von dem Bügelmikrofon (16) aufgefangenen Audiosignalen zu verbessern, wobei die gefilterten Audiosignale zwecks Verringerung eines Okklusionseffekts dem Rechtsohr-Lautsprecher und dem Linksohr-Lautsprecher (30) zugeführt werden, wobei die zentrale Einheit (14) für ein Nutzervorzugslernen ausgebildet ist, um die bevorzugten manuellen Einstellungen durch den Nutzer zu lernen und zu speichern, wobei es sich bei der Audiosignalverarbeitungseinheit (34) um einen einzelnen DSP-Chip handelt, wobei die zentrale Einheit (14) eine Lärmdosimeter-Funktionalität aufweist, wobei die Lärmexposition des Nutzers aus dem Pegel der mittels des Linksohr-Mikrofons und/oder des Rechtsohr-Mikrofons (28) und/oder mittels mindestens eines inneren Mikrofons (31), welches akustisch nach innen zum Gehörgang hin orientiert ist, aufgefangenen Audiosignale abgeschätzt wird und über eine vorgegebene Zeitdauer integriert wird, wobei die zentrale Einheit (14) eine Alarmfunktion aufweist, wobei der Nutzer über den Linksohr-Lautsprecher und/oder den Rechtsohr-Lautsprecher (30) alarmiert wird, wenn eine vorgegebene maximale Lärmdosis erreicht wurde, wobei die zentrale Einheit (14) eine Nutzerschutzfunktion aufweist, wobei die von der Audiosignalverarbeitungseinheit (34) angewandte Verstärkung verringert wird, wenn eine vorgegebene maximale Lärmdosis erreicht wurde, wobei die von der Audiosignalverarbeitungseinheit (34) angewandte Verstärkung, im Umgebungsmodus und/oder im Kommunikationsmodus frequenzabhängig ist, um die Frequenzabhängigkeit der von den Ohrstopfen (10, 12) bewirkten akustischen Dämpfung und/oder einen Gehörverlust des Nutzers zu kompensieren, und wobei die von der Audiosignalverarbeitungseinheit (34) angewandte Verstärkung im Umgebungsmodus und/oder dem Kommunikationsmodus frequenzabhängig ist, um die Sprachverständlichkeit der über den Audioeingang (40, 52) empfangenen Audiosignale zu verbessern.

12. System gemäß einem der vorhergehenden Ansprüche, wobei im Umgebungsmodus einer von einer Mehrzahl von Audiosignalverarbeitungsmodi der Audiosignalverarbeitungseinheit (34) gemäß dem Ergebnis einer Hörumgebungsanalyse automatisch ausgewählt wird.

13. System gemäß einem der Ansprüche 1 bis 11, wobei irs dem Umgebungsmodus ein Audiosignalverarbeitungsmodus aus einer Mehrzahl von Audiosignalverarbeitungsmodi der Audiosignalverarbeitungseinheit (34) auf der zentralen Einheit (14) oder über eine Fernsteuereinheit (18) manuell ausgewählt werden kann.

14. System gemäß einem der vorhergehenden Ansprüche, wobei jeder Ohrstopfen (10, 12) eine individuell angepasste Schale (22) aufweist, in welche die aktive Einheit (24) lösbar eingesetzt ist.

15. Verfahren zum Schützen des Gehörs eines Nutzers, wobei:
ein rechter Ohrstopfen (10) mit einem Mikrofon (28) und einem Lautsprecher (30) mindestens zum Teil im rechten Gehörgang und ein linker Ohrstopfen (12) mit einem Mikrofon (28) und einem Lautsprecher (30) mindestens zum Teil im linken Gehörgang in einer Weise getragen wird, so dass jeder Ohrstopfen für eine akustische Dämpfung von mindestens 10 dB, gemittelt über den hörbaren Frequenzbereich, sorgt;
eine zentrale Einheit (14) mit einer digitalen Audiosignalverarbeitungseinheit (34), welche mindestens zwei Kanäle aufweist, am Körper des Nutzers getragen wird;
eine externe Audiosignalquelle (20) über einen Audioeingang (40, 52) mit der zentralen Einheit (14) verbunden wird;
erfasst wird, ob eine Audiosignalquelle an dem Audiosignaleingang (40, 52) vorhanden ist oder nicht; und
die Audiosignalverarbeitungseinheit (34) in einer Weise gesteuert wird, dass während Zeiten, wenn keine Anwesenheit eines Audiosignals an dem Audioeingang (40, 52) erfasst wird, die Audiosignalverarbeitungseinheit (34) in einem Umgebungsmodus betrieben wird, in welchem die von dem Linksohr-Mikrofon (28) aufgefangenen Audiosignale im Wesentlichen dem Linksohr-Lautsprecher (30) zugeführt werden und die mittels des Rechtsohr-Mikrofons (28) aufgefangenen Audiosignale im Wesentlichen dem Rechtsohr-Lautsprecher (30) zugeführt werden, und während Zeiten, wenn die Anwesenheit eines Audiosignals an dem Audioeingang (40, 52) erfasst wird, die Audiosignalverarbeitungseinheit (34) in einem Kommunikationsmodus betrieben wird, in welchem die mittels des Linksohr-Mikrofons (28) aufgefangenen Audiosignale dem Linksohr-Lautsprecher (30) zugeführt werden und die mittels des Rechtsohr-Mikrofons (28) aufgefangenen Audiosignale dem Rechtsohr-Lautsprecher (30) zugeführt werden, und wobei die über den Audioeingang (40, 52) empfangenen Audiosignale mit den von dem Linksohr-Mikrofon (28) aufgefangenen Audiosignalen und/oder die von dem Rechtsohr-Mikrofon (28) aufgefangenen Audiosignalen gemischt werden, um den LinksohrLautsprecher (30) und/oder dem Rechtsrohr-Lautsprecher (30) zugeführt zu werden, und wobei zumindest ein Parameter, der für die Audiosignalverarbeitung in der Audiosignalverarbeitungseinheit (34) verwendet wird, für den Umgebungsmodus und für den Kommunikationsmodus für mindestens einen der Kanäle unterschiedlich ist, und wobei es sich bei dem Parameter um die Verstärkung als Funktion des Eingangspegels handelt.

## Revendications

1. Système de protection auditive active comprenant un bouchon d'oreille droit (10) devant être porté au moins en partie dans le canal auditif droit d'un utilisateur et un bouchon d'oreille gauche (12) devant être porté au moins en partie dans le canal auditif gauche de l'utilisateur et une unité centrale (14) devant être portée sur le corps de l'utilisateur et comprenant une unité de traitement de signaux audio numériques (34) qui comporte au moins deux canaux et qui peut être connectée via une entrée audio (40) à une source de signaux audio externe (20),
dans lequel chaque bouchon d'oreille (10, 12) est conçu pour assurer une atténuation acoustique d'au moins 10 dB moyennée sur la gamme de fréquences audibles et comprend une unité active (24) comprenant un microphone (28) destiné à capter des signaux audio provenant d'un son ambiant et d'un haut-parleur (30) qui sont connectés à l'unité de traitement de signaux audio (34),
dans lequel l'unité centrale comprend un détecteur (56) destiné à détecter la présence d'un signal audio sur l'entrée audio (40, 52),
et dans lequel l'unité centrale (14) est conçue pour commander l'unité de traitement de signaux audio (34) de manière à ce que, aux instants où la présence d'un signal audio sur l'entrée audio (40, 52) n'est pas détectée, l'unité de traitement de signaux audio (34) est mise en fonctionnement dans un mode ambiant dans lequel les signaux audio captés par le microphone d'oreille gauche (28) sont principalement fournis au haut-parleur d'oreille gauche (30) et les signaux audio captés par le microphone d'oreille droite (28) sont principalement fournis au haut-parleur d'oreille droite (30), et aux instants où la présence d'un signal audio sur l'entrée audio (40, 52) est détectée, l'unité de traitement de signaux audio (34) est mise en fonctionnement dans un mode de communication dans lequel les signaux audio captés par le microphone d'oreille gauche (28) sont fournis au haut-parleur d'oreille gauche (30) et les signaux audio captés par le microphone d'oreille droite (28) sont fournis au haut-parleur d'oreille droite (30), dans lequel les signaux audio reçus via l'entrée audio (40, 52) sont mélangés à au moins l'un des signaux audio captés par le microphone d'oreille gauche (28) et des signaux audio captés par le microphone d'oreille droite (28) afin qu'ils soient fournis à au moins l'un du haut-parleur d'oreille gauche (30) et du haut-parleur d'oreille droite (30), et dans lequel au moins un paramètre utilisé pour le traitement de signaux audio dans l'unité de traitement de signaux audio (34) est différent pour le mode ambiant et le mode de communication pour au moins l'un des canaux, et dans lequel le paramètre est le gain en fonction du niveau d'entrée.

2. Système selon la revendication 1, dans lequel le rapport de mélange est différent pour le mélange avec les signaux audio captés par le microphone d'oreille gauche (28) et pour le mélange avec les signaux audio captés par le microphone d'oreille droite (28) .

3. Système selon l'une des revendications précédentes, dans lequel, à la fois dans le mode ambiant et dans le mode de communication pour les deux canaux de l'unité de traitement de signaux audio (34), le çain est progressivement réduit au-dessus d'une valeur de seuil donnée du niveau d'entrée, dans lequel le gain est sensiblement indépendant du niveau d'entrée pour des niveaux d'entrée inférieurs à la valeur de seuil, dans lequel, pour des niveaux d'entrée inférieurs à la valeur de seuil, le gain d'au moins l'un des canaux peut être ajusté par une commande de volume manuelle (18), dans lequel, dans le mode ambiant, le gain des deux canaux peut être ajusté en parallèle par la commande de volume manuelle (18), dans lequel, dans le mode de communication, seul le gain du canal acheminant principalement les signaux audio captés par au moins l'un du microphone d'oreille gauche (28) et du microphone d'oreille droite (28) peut être ajusté par la commande de volume manuelle (18), dans lequel, dans le mode de communication, le niveau de sortie acoustique maximum est différent pour les deux canaux, dans lequel, dans le mode ambiant, la fonction de gain de l'unité de traitement de signaux audio (34) peut être sélectionnée manuellement ou est automatiquement sélectionnée sur la base du résultat d'une analyse de scène auditive, dans lequel l'unité centrale (14) comprend une interface bidirectionnelle avec un dispositif de communication sans fil (20), lequel dispositif de communication est destiné à fournir des signaux audio à l'entrée audio (40, 52), dans lequel le système comprend un moyen (16, 28) destiné à capter des signaux audio devant être délivrés au dispositif de communication (20) et dans lequel le système comprend une unité de commande à distance (18) apte à effectuer la commande manuelle du dispositif de communication (20), dans lequel l'unité centrale (14) comprend une interface bidirectionnelle avec un dispositif de communication sans fil (20), lequel dispositif de communication est destiné à fournir des signaux audio à l'entrée audio (40, 52), dans lequel le système comprend un moyen (16, 28) destiné à capter des signaux audio devant être délivrés au dispositif de communication (20) et dans lequel l'unité centrale (14) comprend une interface utilisateur pour la commande manuelle du dispositif de communication (20), et dans lequel l'interface bidirectionnelle est sans fil.

4. Système selon la revendication 3, dans lequel le moyen destiné à capter des signaux audio est un microphone monté sur tige (16) qui doit être porté par l'utilisateur afin de capter la voix de l'utilisateur et qui est électriquement connecté au dispositif de communication directement ou via ladite interface.

5. Système selon la revendication 3, dans lequel le moyen destiné à capter des signaux audio est au moins l'un du microphone d'oreille gauche (28) et du microphone d'oreille droite (28) et/ou au moins un microphone intérieur (31) acoustiquement orienté vers l'intérieur dans la direction du canal auditif.

6. Système selon la revendication 5, dans lequel une unité BSS est contenue dans l'unité de traitement de signaux audio (34) afin de faire en sorte que les signaux audio soient captés par au moins l'un du microphone d'oreille gauche (28) et du microphone d'oreille droite (28) et/ou qu'au moins un microphone intérieur (31) soit soumis à un algorithme BBS afin de séparer la voix de l'utilisateur d'un son ambiant.

7. Système selon la revendication 5, dans lequel l'unité de traitement de signaux audio (34) est capable d'effectuer une mise en forme de faisceau sur les signaux audio captés par le microphone d'oreille gauche (28) et le microphone d'oreille droite (28) afin de séparer la voix de l'utilisateur d'un son ambiant, et dans lequel l'unité de commande à distance (18) est une unité portative connectée à l'unité centrale (34) par une liaison sans fil.

8. Système selon la revendication 3, dans lequel l'unité centrale (14) et/ou l'unité de commande à distance (18) comprend un élément à poussoir de conversation (35) destiné à commander la transmission des signaux audio délivrés par le moyen de capture de signaux audio (16, 28, 31) par le dispositif de communication (20).

9. Système selon la revendication 3, dans lequel l'unité de commande à distance (18) comprend un élément de commande du volume destiné à commander le gain appliqué par l'unité de traitement de signaux audio (34).

10. Système selon la revendication 3, dans lequel le dispositif de communication (20) est un téléphone mobile, et dans lequel l'unité de commande à distance (18) comprend un élément d'acceptation d'appel/rejet d'appel et un élément d'activation de numérotation vocale.

11. Système selon la revendication 3, dans lequel le dispositif de communication (20) est un téléphone mobile, et dans lequel l'unité centrale (14) comprend un élément d'acceptation d'appel/rejet d'appel et un élément d'activation de numérotation vocale, dans lequel de l'énergie est délivrée du dispositif de communication (20) à l'unité centrale (14) via l'interface bidirectionnelle, dans lequel l'unité de traitement de signaux audio (34) dans le mode ambiant est conçue pour améliorer par filtrage la voix de l'utilisateur dans les signaux audio captés par le microphone d'oreille gauche (28) et le microphone d'oreille droite (28) ou dans les signaux audio captés par un microphone monté sur tige (16), les signaux audio filtrés étant fournis aux haut-parleurs d'oreille droite et d'oreille gauche (30) afin de réduire l'effet d'occlusion, dans lequel l'unité centrale (14) est capable d'effectuer un apprentissage des préférences de l'utilisateur afin d'apprendre et de stocker les réglages manuels préférés de l'utilisateur, dans lequel l'unité de traitement de signaux audio (34) est une puce DSP unique, dans lequel l'unité centrale (14) comprend une fonctionnalité de dosimétrie du bruit, l'exposition sonore de l'utilisateur étant estimée à partir du niveau des signaux audio captés par le microphone d'oreille gauche et/ou d'oreille droite (28) et/ou par au moins un microphone intérieur (31) acoustiquement orienté vers l'intérieur dans la direction du canal auditif et intégré sur une période de temps donnée, dans lequel l'unité centrale (14) comprend une fonction d'alarme au moyen de laquelle l'utilisateur est alerté via au moins l'un des haut-parleurs d'oreille gauche et d'oreille droite (30) lorsqu'une dose de bruit maximale donnée a été atteinte, dans lequel l'unité centrale (14) comprend une fonction de protection de l'utilisateur au moyen de laquelle le gain appliqué dans l'unité de traitement de signaux audio (34) est réduit lorsqu'une dose de bruit maximale donnée a été atteinte, dans lequel le gain appliqué par l'unité de traitement de signaux audio (34) dans au moins l'un du mode ambiant et du mode de communication dépend de la fréquence afin de compenser au moins l'une de la dépendance vis-à-vis de la fréquence de l'atténuation acoustique produite par les bouchons d'oreille (10, 12) et d'une perte d'audition de l'utilisateur, et dans lequel le gain appliqué par l'unité de traitement de signaux audio (34) dans au moins l'un du mode ambiant et du mode de communication dépend de la fréquence afin d'améliorer l'intelligibilité de la parole des signaux audio reçus via l'entrée audio (40,52).

12. Système selon l'une des revendications précédentes, dans lequel, dans le mode ambiant, l'un d'une pluralité de modes de traitement de signaux audio de l'unité de traitement de signaux audio (34) est automatiquement sélectionné conformément au résultat d'une analyse de scène auditive.

13. Système selon l'une des revendications 1 à 11, dans lequel, dans le mode ambiant, l'un d'une pluralité de modes de traitement de signaux audio de l'unité de traitement de signaux audio (34) peut être manuellement sélectionné sur l'unité centrale (14) ou via une unité de commande à distance (18).

14. Système selon l'une des revendications précédentes, dans lequel chaque bouchon d'oreille (10, 12) comprend une coque personnalisée (22) dans laquelle l'unité active (24) est insérée de manière amovible.

15. Procédé destiné à fournir une protection auditive à un utilisateur, consistant à :
porter un bouchon d'oreille droit (10) comprenant un microphone (28) et un haut-parleur (30) au moins en partie dans le canal auditif droit et un bouchon d'oreille gauche (12) comprenant un microphone (28) et un haut-parleur (30) au moins en partie dans le canal auditif gauche de manière à ce que chaque bouchon d'oreille assure une atténuation acoustique d'au moins 10 dB moyennée sur la gamme de fréquences audibles ; porter une unité centrale (14) comprenant une unité de traitement de signaux audio numériques (34) qui comporte au moins deux canaux sur le corps de l'utilisateur ;
connecter une source de signaux audio externe (20) via une entrée audio (40, 52) à l'unité centrale (14) ;
détecter si un signal audio est ou non présent sur l'entrée audio (40, 52) ; et
commander l'unité de traitement de signaux audio (34) de manière à ce que, aux instants où la présence d'un signal audio sur l'entrée audio (40, 52) n'est pas détectée, l'unité de traitement de signaux audio (34) soit mise en fonctionnement dans un mode ambiant dans lequel les signaux audio captés par le microphone d'oreille gauche (28) sont principalement fournis au haut-parleur d'oreille gauche (30) et les signaux audio captés par le microphone d'oreille droite (28) sont principalement fournis au haut-parleur d'oreille droite (30), et qu'aux instants où la présence d'un signal audio sur l'entrée audio (40, 52) est détectée, l'unité de traitement de signaux audio (34) soit mise en fonctionnement dans un mode de communication dans lequel les signaux audio captés par le microphone d'oreille gauche (28) sont fournis au haut-parleur d'oreille gauche (30) et les signaux audio captés par le microphone d'oreille droite (28) sont fournis au haut-parleur d'oreille droite (30), et dans lequel les signaux audio reçus via l'entrée audio (40, 52) sont mélangés à au moins l'un des signaux audio captés par le microphone d'oreille gauche (28) et les signaux audio captés par le microphone d'oreille droite (28) afin qu'ils soient fournis à au moins l'un du haut-parleur d'oreille gauche (30) et du haut-parleur d'oreille droite (30), et dans lequel au moins un paramètre utilisé pour le traitement de signaux audio dans l'unité de traitement de signaux audio (34) est différent pour le mode ambiant et le mode de communication pour au moins l'un des canaux, et dans lequel le paramètre est le gain en fonction du niveau d'entrée.
